# EUROPEAN PATENT APPLICATION

(11) **EP 0 599 183 A1**
(43) Date of publication of application: **01.06.1994**
(21) Application number: 93118486.5
(22) Date of filing: 16.11.1993
(51) Int. Cl.: C07D 233/84, C07C 255/25, C07C 327/42

(54) **Manufacturing of pesticides and intermediates**

(30) Priority: 25.11.1992 US 981584; 03.03.1993 US 25503
(71) Applicant: RHONE-POULENC AGROCHIMIE, 69009 Lyon (FR)
(72) Inventor: Aubert, Thierry, F-64000 Pau (FR); Chene, Alain, Les Terrasses de Saint Rambert, F-69009 Lyon (FR)

(57) **Abstract**

The present invention relates to novel 1-aryl-imidazol-4-yl thiols derivatives, in particular to 1-aryl-4-mercaptoimidazoles N-carbonyl anilines which are intermediates for the preparation of pesticidal 1-aryl imidazoles and to processes for making them.

## Description

### BACKGROUND OF THE INVENTION

### 1. Field of the invention

The present invention relates to novel 1-aryl-imidazol-4-yl thiols derivatives, to intermediates and processes to make these compounds. The 1-aryl-imidazol-4-yl thiols derivatives of the present invention are useful and valuable intermediates for the preparation of 1-aryl imidazoles useful as pesticides for controlling arthropod, nematode, helminth or protozoan pests and more particularly for controlling arthropods, especially mites or foliar or soil insects, without causing injury to crop plants.

### 2. Description of the Related Art

Various substituted imidazole compounds are known to exhibit a wide variety of pesticidal activities including activity as herbicides, plant growth regulators, fungicides, nematicides, insecticides and biocides. Included are the following:
WO Pat. No. 9100-277-A discloses, as angiotensin II blockers, 1-arylimidazoles , which may be substituted at the 4 or the 5 position of the imidazole ring by a thiol group. The two remaining positions on the imidazole ring are substituted and the phenyl ring cannot be substituted by three halogens.

European Patent Application No. EP 349-763-A discloses, as part of topical pharmaceutical compositions, some imidazoles, which may be substituted at the 1 position of the imidazole ring by an aryl group and by a thiol function at a non-specified position.

Japanese Patent Application No. J6 3239-273-A discloses, as drugs improving cerebral function or acting on amnesia, 1-arylimidazoles, which may be substituted at the 4 position of the imidazole ring by a thiol group; one of the two positions 4 or 5 of the imidazole ring bears a "X-aryl" function, X being a methylene or a carbonyl group.

Various substituted N-carbonyl anilines are known to be useful as intermediates of biologically active material.

US patents 3200151 and 3131218 describe N-cyanomethyl N-formyl 2,6-dimethylaniline as intermediates of pharmaceuticals.

### SUMMARY OF THE INVENTION

It is an objective of the present invention to provide novel 1-aryl-imidazol-4-yl thiols derivatives.

A further objective of the present invention is to provide a new process to achieve the preparation of N-carbonyl anilines which are further N-substituted by means of a radical cyano methylene or carbothioamide methylene.

A further objective of the present invention is to provide a new process to achieve the preparation of pesticidal 1-aryl imidazoles.

These and other objectives of the invention shall become readily apparent from the detailed description of the present invention.

Therefore the present invention provides novel 1-aryl-imidazol-4-yl thiols derivatives (including their stereoisomers, resulting from a either optical or geometrical isomery) of the general formula **I**: 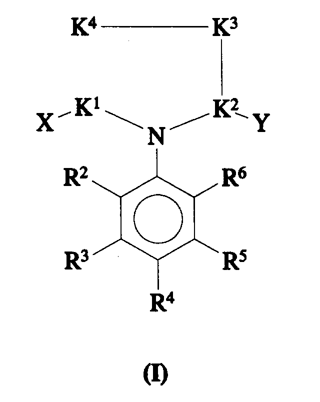
wherein:
- K¹ represents -CO-, or 〉C= ,K¹ being connected to K⁴ through the double free bond when K¹ is 〉C=
- K² represents 〉C= the double free bond being connected to K³, or K² is 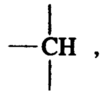 and
- K³ represents: 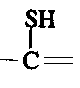 or ≡C- or -CS-, and
- K⁴ represents:
   ⁻ -N= when K¹ represents 〉C= , K⁴ being then connected both with K³ and K¹ through the covalent bond ,
   ⁻ or N≡ or NH₂-, K⁴ being then connected only to K³ through one covalent bond,
the link between K⁴ and K³ being either a monovalent bond or a trivalent bond according to the specific meaning of K⁴ and K³ respectively,
the link between K³ and K² being either a monovalent bond or a divalent bond according to the specific meaning of K³ and K² respectively,
- X, Y, R², R³, R⁴, R⁵ and R⁶ are each individually selected from : a hydrogen or a halogen atom or a cyano group or a C₁ to C₄ straight chain or branched chain alkyl, which group is unsubstituted or substituted by one or more halogen atoms, which are the same or different, up to full substitution; and
   provided that R² and R⁶ are not simultaneously a methyl when:
   . X and Y represent a hydrogen atom, and
   . K² is 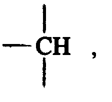 and K³ is ≡C- , and K⁴ is N≡
Of course the meaning of the substituents in formula (I) are chosen respectively in such a way that the number and position of mono- or di- or trivalent bonds fit together adequately. The HS-C=C group may also be in the non enolic form -C(S)-CH- and vice-versa.

A preferred family of the compounds according to general formula I is represented by the compounds of general formula (Ia): 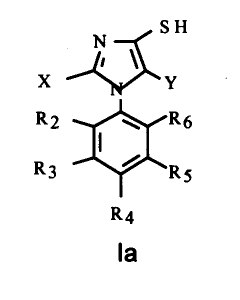
wherein:
X, Y, R², R³, R⁵ and R⁶ are each individually selected from: a hydrogen or a halogen atom or a C₁ to C₄ straight chain or branched chain alkyl, which group is unsubstituted or substituted by one or more halogen atoms, which are the same or different, up to full substitution; and
R⁴ is selected from: a hydrogen or a halogen atom or a cyano group or an unsubstituted C₁ to C₄ alkyl or C₁ to C₄ alkyl substituted by one or more halogen atoms, which are the same or different, up to full substitution.

Another preferred family of the compounds according to general formula I is represented by the compounds of general formula (Ib): 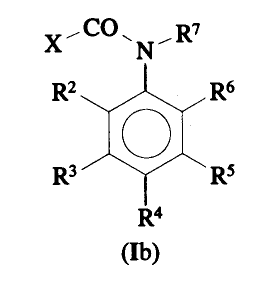
wherein:
X is a hydrogen atom or a C₁ to C₄ straight chain or branched chain alkyl group, which group is unsubstituted or substituted by one or more halogen atoms which are the same or different, up to full substitution of the alkyl moiety; and
R², R³, R⁴, R⁵, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety ; and
R⁷ represents a group -CHY-CN or -CHY-CS-NH₂, in which Y is a hydrogen atom or a C₁ to C₄ straight chain or branched chain alkyl group
provided that R² and R⁶ are not simultaneously a methyl when X and R⁷ represent, respectively, a hydrogen atom and a CH₂-CN group.

According to a preferred feature of the invention, the 1-aryl-imidazol-4-yl thiols derivatives of general formula I are selected from amongst the compounds of the formula (II): 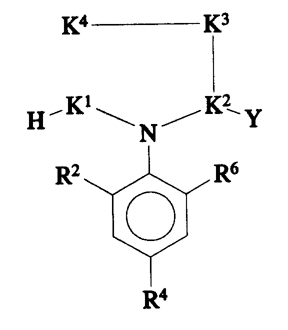
wherein:
- K¹ represents -CO-, or 〉C= ,K¹ being connected to K⁴ through the double free bond when K¹ is 〉C=
- K² represents 〉C= the double free bond being connected to K³, or K² is 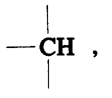 and
- K³ represents : 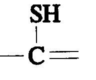 or ≡C or -CS- , and
- K⁴ represents :
   - -N= when K¹ represents 〉C= , K⁴ being then connected both with K³ and K¹ through the covalent bond,
   - or N≡ or NH₂-, K⁴ being then connected only to K³ through one covalent bond,
the link between K⁴ and K³ being either a monovalent bond or a trivalent bond according to the specific meaning of K⁴ and K³ respectively,
the link between K³ and K² being either a monovalent bond or a divalent bond according to the specific meaning of K³ and K² respectively,
Y is a hydrogen atom or methyl; and
R², R⁴, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety ; and
provided that R² and R⁶ are not simultaneously a methyl when:
. Y represents a hydrogen atom, and
. K² is 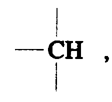 and K³ is ≡C- , and K⁴ is N≡
According to a further preferred feature of the invention, the 1-aryl-imidazol-4-yl thiols derivatives of general formula I are selected from amongst the compounds of the formula (IIa): 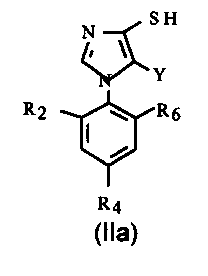
wherein:
Y is a hydrogen atom or methyl; and
R² and R⁶ are a hydrogen or a halogen atom or methyl; and
R⁴ is a halogen atom.

According to a further preferred feature of the invention, the 1-aryl-imidazol-4-yl thiols derivatives of general formula I are selected from amongst the compounds of the formula (IIb): 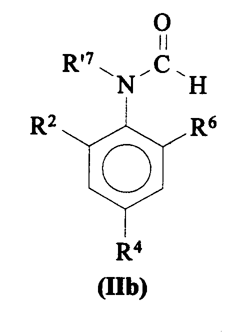
wherein:
R², R⁴, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety;
R'⁷ represents a group -CH₂-CN or -CH₂-CS-NH₂;
provided that R² and R⁶ are not simultaneously a methyl when R'⁷ represents a CH₂-CN group.

According to a further preferred feature of the invention, the new compounds of the invention are selected from amongst the compounds of formula **IIb** wherein:
R² and R⁶ are a halogen or a hydrogen, and
R⁴ is a halogen.

The novel compounds of the present invention are useful for the preparation of 1-aryl imidazoles which are known to exhibit pesticidal properties, especially as insecticides or miticides or both, such as the following compounds of formula **III**: 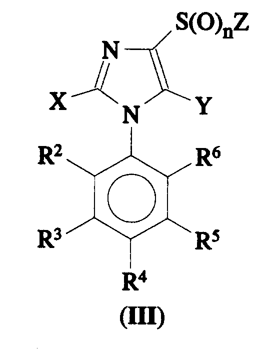
wherein:
X, Y, R², R³, R^{5,} and R^{6,} are each individually selected from: a hydrogen or a halogen atom or a C₁ to C₄ straight chain or branched chain alkyl, which group is unsubstituted or substituted by one or more halogen atoms, which are the same or different, up to full substitution of the alkyl moiety; and
Z is a C₁ to C₄ straight chain or branched chain alkyl, which group is unsubstituted or substituted by one or more halogen atoms, which are the same or different, up to full substitution; and
n is 0, 1 or 2; and
R⁴ selected from: a hydrogen or a halogen atom or a cyano group or a unsubstituted C₁ to C₄ alkyl or C₁ to C₄ alkyl substituted by one or more halogen atoms, which are the same or different, up to full substitution.

Such pesticides are described for example in the European patent applications EP 0 396427 and EP 0 484165.

### DESCRIPTION OF THE PREFERRED EMBODIMENTS

### A) Methods or processes of synthesis of the compounds of formula Ia:

The new and novel 1-aryl-4-mercaptoimidazoles of this invention, namely 4 in Scheme I below, can be conveniently prepared by the following method, in which X, Y, and R² to R⁶ are as defined in formula (Ia). 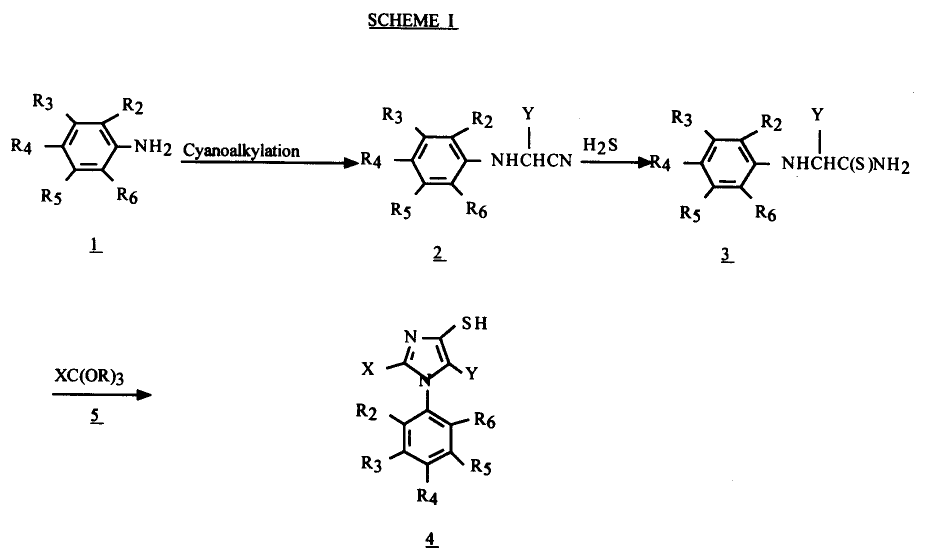
In Scheme I the starting materials, the anilines 1, are generally commercially available or may be prepared following well known literature procedures.

Some of the N-cyanoalkylaniline intermediates 2 are known compounds which, however, are not commercially available. Others of the intermediates 2 have not been reported previously in the literature. All of these substituted N-cyanoalkylaniline compounds may be prepared according to the following literature procedures:
a) A N-cyanoalkylaniline intermediate 2 may be obtained by direct alkylation with an appropriate alkyl halide, such as iodoacetonitrile, bromoacetonitrile, chloroacetonitrile, 2-bromopropionitrile, etc., of a substituted aniline of the general formula 1 in a suitable solvent, such as acetone, acetonitrile, alcohol, dimethylformamide,etc., in the presence of a base, such as sodium or potassium hydroxide, carbonate, tertiary amine etc., at a reaction temperature from about 50 to about 200°C, preferably at a temperature from about 80 to about 150°C. Representative procedures are given in P. R. Marsham et al., J. Med. Chem., 1990, 33, 3072, in T. R. Jones et al., J. Med. Chem., 1985, 28, 1468, and in M. G. Nair et al., J. Med. Chem., 1983, 26, 605.
b) A N-cyanoalkylaniline intermediate 2 may be obtained by reacting a substituted aniline of the general formula 1 with the bisulfite addition product of an aldehyde, such as formaldehyde, acetaldehyde, propionaldehyde, isobutyraldehyde, etc., in an aqueous reaction medium, followed by addition of a aqueous appropriate cyanide, such as, for example, a alkali metal or earth alkaline metal cyanide, e. g. sodium cyanide or potassium cyanide, at a reaction temperature from about 20 to about 110°C, preferably at a temperature from about 60 to about 100°C. The intermediate 2 may also be prepared by reacting a substituted aniline of the general formula 1 with an aldehyde defined as above, in the presence of a appropriate cyanide defined as above. Said reaction is conveniently carried out in the presence of a suitable relatively polar inert reaction solvent such as, for example, glacial acetic acid, water and the like, or a mixture of two or more relatively polar solvents, at a temperature defined as above. When the aniline 1 is a weekly basic amine, it is advantageous to carry out the reaction in the presence of a anhydrous zinc halide, e. g. zinc chloride, in a relatively polar anhydrous solvent, e. g. glacial acetic acid. Representative procedures for this Strecker synthesis are reported in J. March, "Advanced Organic Chemistry", Third Ed., 1985, Wiley-Interscience, p. 855 and in references therein, in G. N. Walker et al., J. Org. Chem., 1972, 37, 3755, and in A. Takeda, J. Org. Chem., 1957, 22, 1096.
c) A N-cyanoalkylaniline intermediate 2, in which Y has all the meanings given in the definition of formula (Ia) but a hydrogen atom and the other groups are as defined above, may also be obtained by a slightly different procedure from b), which consists of reacting a substituted aniline of the general formula 1 with a aldehyde cyanohydrin (which is generally commercially available or may be prepared following well known literature procedures), such as acetaldehyde cyanohydrin (also called lactonitrile), propionaldehyde cyanohydrine, etc., in a suitable solvent, such as methanol, ethanol etc., at a reaction temperature from about 20 to about 120°C, preferably at a temperature from about 60 to about 80°C. Representative procedures are reported in J. Huang and D. S. Haigh, J. Heterocyclic Chem., 1990, 27, 331 and in W. E. McEwen et al., J. Org. Chem., 1980, 45, 1301.
d) A N-cyanoalkylaniline intermediate 2, in which R² and/or R⁶ are/is a halogen atom and the other groups are as defined above, may be prepared by halogenating a N-cyanoalkylaniline intermediate 2, in which R² and/or R⁶ are/is a hydrogen atom and the other groups are as defined above, with a halogenating agent, such as sulfuryl chloride, thionyl chloride, chlorine or bromine, N-chlorosuccinimide or N-bromosuccinimide, etc.. The reaction is conducted in an inert aprotic solvent, such as a chlorinated hydrocarbon, an ether, acetonitrile, etc. The reaction may be carried out between about -50°C and 150°C, preferably between about -10°C and 100°C, depending on the reactivity of the intermediate 2 and the reactivity of the halogenating agent used. If so the ortho substituents R² and/or R⁶ are introduced following formation of the nitrile 2. A representative procedure is reported in US Patent No 4,226,802.

Carbothioamide intermediates 3 are known compounds which, however, are not commercially available. Others of the intermediates 3 have not been reported previously in the literature. Carbothioamide 3 may be prepared by reacting a N-cyanoalkylaniline intermediate 2 with gaseous hydrogen sulfide in a suitable inert solvent, such as methanol, ethanol, dimethyl sulfoxide, dimethylformamide, pyridine and the like, etc., in the presence of a appropriate base, such as ammonia, a trialkylamine e.g. triethylamine, etc., at a reaction temperature from about -20 to about 100°C, preferably at a temperature from about -10 to about 50°C. Representative procedures are reported in L. Cassar, S. Panossian and C. Giordano, Synthesis, 1978, 917 and in references therein.

The 1-aryl-4-mercaptoimidazoles 4 are new compounds, which can be used to produce the known compounds of Formula III. A carbothioamide intermediate 3 may be cyclized to the corresponding 1-aryl-4-mercaptoimidazole 4 by reacting with a alkyl orthoformate of the general formula 5, XC(OR)₃, in which R is a C₁ to C₄ alkyl group and X is defined as above. This alkyl orthoformate is generally commercially available or may be prepared following well known literature procedures. Said reaction is conveniently carried out in the presence of a suitable reaction inert solvent, such as toluene and the like, chlorobenzene and the like, dimethylformamide, dimethylsulfoxide and the like, or preferably without any solvent, at a reaction temperature from about 20 to about 200°C, preferably at a temperature from about 50 to about 150°C. Starting from 1 equivalent of the carbothioamide 3, the amount of the alkyl orthoformate 5 used varies from 1 equivalent to a large excess, preferably a large excess (if so the alkylorthoformate 5 itself is used as solvent for the reaction). The alcohol of the general formula ROH produced during the reaction may or may not be distilled as soon as it is formed, for example by or by not conducting the reaction under reduced pressure, from about 5 to 200 torrs, preferably from about 10 to about 100 torrs. The compounds 4 are highly reactive and oxidize rapidly to the corresponding disulfides in the presence of air. Therefore, they may be used directly in Scheme III without further treatment.

### B) Methods or processes of synthesis of the compounds of formula Ib:

The general manufacturing process of the novel N-carbonyl anilines of formula Ib, namely 8 and 9, is described in the Scheme II below, in which the substituents of the various compounds are those defined in the general formula (Ib). 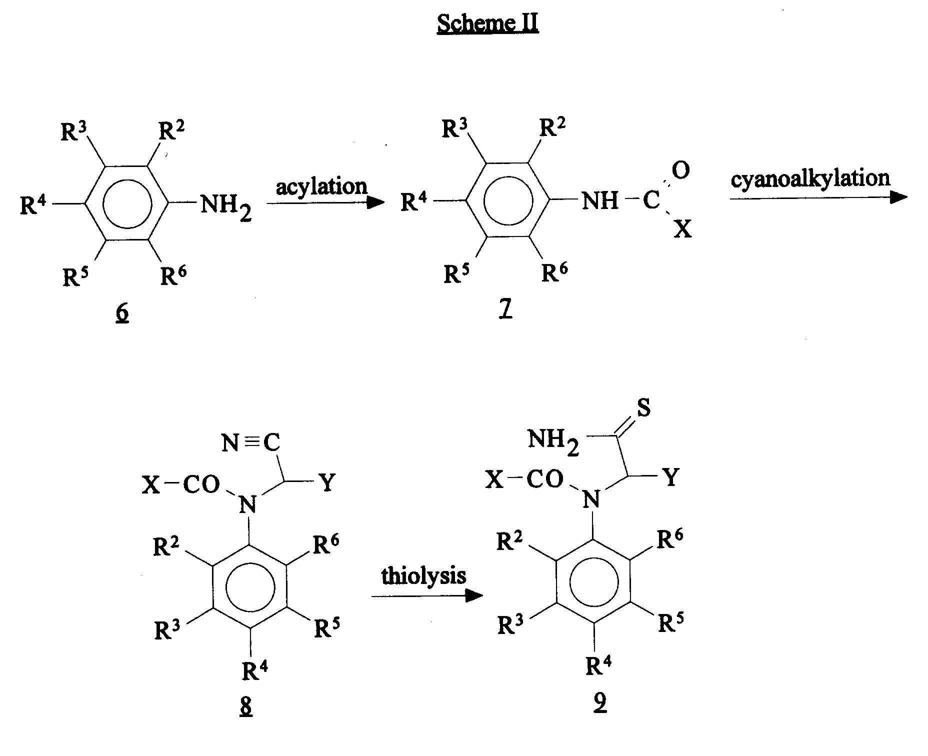
In Scheme II the starting materials, the uncarbonylated anilines 6, are generally commercially available or may be prepared following well known literature procedures. Well known procedures may be found by the man skilled in the art in general Chemistry Handbooks as well as in the literature identified in the Chemical Abstracts.

In a first step, the manufacturing process of the N-carbonylated anilines of formula 7, starting from the compounds of formula 6, is described hereafter.

When X represents a C₁ to C₄ straight chain or branched chain alkyl group, which group is unsubstituted or substituted by one or more halogen atoms which are the same or different, up to full substitution of the alkyl moiety, the N-carbonylated anilines 7 may be prepared by reacting in a liquid medium a substituted aniline of the general formula 6 with anhydrides of lower alkanoic acids of formula (10):

(X-CO)₂O (10)

The specific anhydride is chosen according to the exact formula of the anilines which is sought.

When X represents a hydrogen, the N-carbonylated anilines 7 may be prepared by reacting a substituted aniline of the general formula 6 with the anhydride of formula: 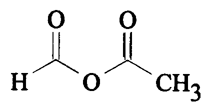
according to the methods described in Tet. Lett. 1982, 23, 3315 and J. Org. Chem. 1958, 23, 727 or similar methods adapted by the man skilled in the art choosing adequately the substituents.

In a second step, the manufacturing process of the compounds of formula 8, starting from the compounds of formula 7, is described.

The cyano derivatives of N-carbonylated anilines, of formula 8 in the Scheme II in which X, Y, and R² to R⁶ are as defined in general formula Ib, are novel compounds, provided that R² and R⁶ are not simultaneously a methyl when X and Y both represent a hydrogen atom. They may be prepared from the corresponding N-carbonylated anilines, of formula 7, by cyanoalkylation, that is to say by reacting a compound of formula (11):

Σ-CHY-CN (11)

wherein:
Σ is a halogen, hydroxyde, alkylcarbonyloxy, haloalkylcarbonyloxy, arylcarbonyloxy, alkylsulfonyloxy, haloalkylsulfonyloxy, arylsulfonyloxy, preferably phenylsulfonyloxy;
Y has the same definition as in formula Ib.

This reaction is carried out in a biphasic reaction medium in conditions known as liquid-liquid phase transfer conditions. This means that there is an organic liquid phase and an aqueous liquid phase with transfer of reactants and/or catalyst from one phase to the other.

Some of the compounds of formula 11 are commercial, others can be prepared by methods known by the man skilled in the art.

When Σ is a phenylsulfonyloxy, the preparation of the compound of formula 11 may be done according to the method described in Acta Pol. Pharm. 23 (5), p. 417 (1966). In this case, the compound of formula 11 may be implemented in the cyanoalkylation reaction without being isolated or purified : the aqueous phase in which this compound is obtained can be used as the aqueous liquid phase of the cyanoalkylation reaction.

The organic phase for the cyanoalkylation reaction is mainly an organic solvent wherein the anilines are soluble. Advantageously, this solvent may be an aliphatic or aromatic hydrocarbon which may be halogenated, or a derivative of such compounds having an oxygen atom therein which forms an ether bond. Preferred organic solvents are chlorinated hydrocarbons such as dichloromethane, or aromatic hydrocarbons such as toluen or monochlorobenzene.

The aqueous liquid phase comprises a water soluble base and a phase transfer catalyst. The water soluble base may be organic or inorganic, and is preferably an hydroxide or carbonate of alkaline or earth-alkaline metal. The liquid-liquid phase transfer catalysts are well known ; alkylammonium halides are preferred. The ratio : catalyst / starting material 7 ( defined as mole number/mole number), is generally between 0.02 and 0.4, preferably between 0.05 and 0.25. The reaction temperature is generally between -10 and 50°C, preferably between 5 and 20°C (room temperature).

In a third step, the manufacturing process of the compounds of formula 9, starting from the compounds of formula 8, is now described.

The carbothioamide methylene substituted N-carbonylated aniline, of formula 9 in the Scheme II in which X, Y and R² to R⁶ are as defined in general formula Ib, are novel compounds which may be prepared by thiolysis of the cyano derivatives of N-carbonylated anilines of formula 8 in the Scheme II. This reaction of thiolysis is a reaction known per se, and any procedure known to thiolyze a nitrile group into an amino thiocarbonyl group may be used, such as:
* action of esters of the dithiophosphoric acid as described in J. Heterocyclic Chem. 28, 1607 (1991); or
* action of phosphorus polysulfide, preferably decasulfide, in presence of an alkaline sulfide, as described in Synth. Commun., 22 (10), 1397 (1992).

The preferred procedure for thiolysis involves a base-catalyzed action of hydrogen sulfide (gas) under either gas-liquid or gas-liquid-liquid phase transfer conditions. The reaction may be carried out under an hydrogen sulfide pressure comprised in the range from 1 to 4 bar, preferably from 1 to 2 bar. The ratio : mole number of hydrogen sulfide / mole number of cyano derivatives 8, is generally in the range from 1 to 10, preferably from 1 to 2. The reaction temperature is generally in the range from 25 to 80°C, preferably from 30 to 40°C.

In the case of a gas-liquid system, the liquid phase may be a polar organic solvent wherein the cyano derivatives 8 are soluble. Advantageously, the solvent may be a protic solvent such as an alcohol or an aprotic solvent such as dimethylformamid, dimethylsulfoxid, sulfolane, acetone, or pyridine. The catalytic base may be an amine (as described in J. Chem. Eng. Data, 13, 130 (1968)), an hydroxide or alkoxide of alkaline or earth-alkaline metal, a basic ion-exchange resin, an alkylammonium hydroxide, an hydrosulphide of alkaline metal, or sodium sulphide. Preferred bases are aromatic amines such as pyridin or dimethylaminopyridin, primary, secondary or tertiary aliphatic amines including alkanolamines, such as ethanolamine and diethanolamine, tetra-(lower alkyl) guanidines, ammonia, or a mixture of amines such as a mixture thereof, preferably triethylamine and pyridine. The ratio : concentration of catalytic base/concentration of cyano derivatives 8 (defined as mole number / mole number ) is generally between 0 and 3, preferably between 0.8 and 1.5.

In the case of a gas-liquid-liquid system, such as described in Synthesis, 917 (1978), one liquid phase is an organic one which is mainly an aliphatic or aromatic hydrocarbon, which may be halogenated, or an ether. Preferred organic solvents are chlorinated aromatic hydrocarbon, such as dichlorobenzene, or an ether such as diphenyl ether. The other liquid phase is an aqueous liquid phase which comprises a phase transfer catalyst and sodium sulphide. Preferred phase transfer catalyst are tetra-alkylammonium halides and the ratio : mole number of catalyst/ mole number of starting material 8 is generally berween 0.01 and 0.1. The ratio : mole number of sodium sulphide / mole number of starting material 8 is generally between 0.02 and 0.2.

### C) Methods or processes of synthesis of compounds of formula III:

Scheme III herebelow now describes, starting from the novel compounds according to the invention, of formula **Ib**, namely of formula 9, or of formula Ia, namely of formula 4, the general manufacturing process of some of the 1-aryl imidazoles of formula **III**, namely the 1-aryl imidazoles of formula 14, wherein the definitions of X, Y, R², R³, R⁴, R⁵, R⁶ are those given in the general formula Ib, and R⁷ and R⁸ represent F, Cl, Br or a perfluoroalkyl group. 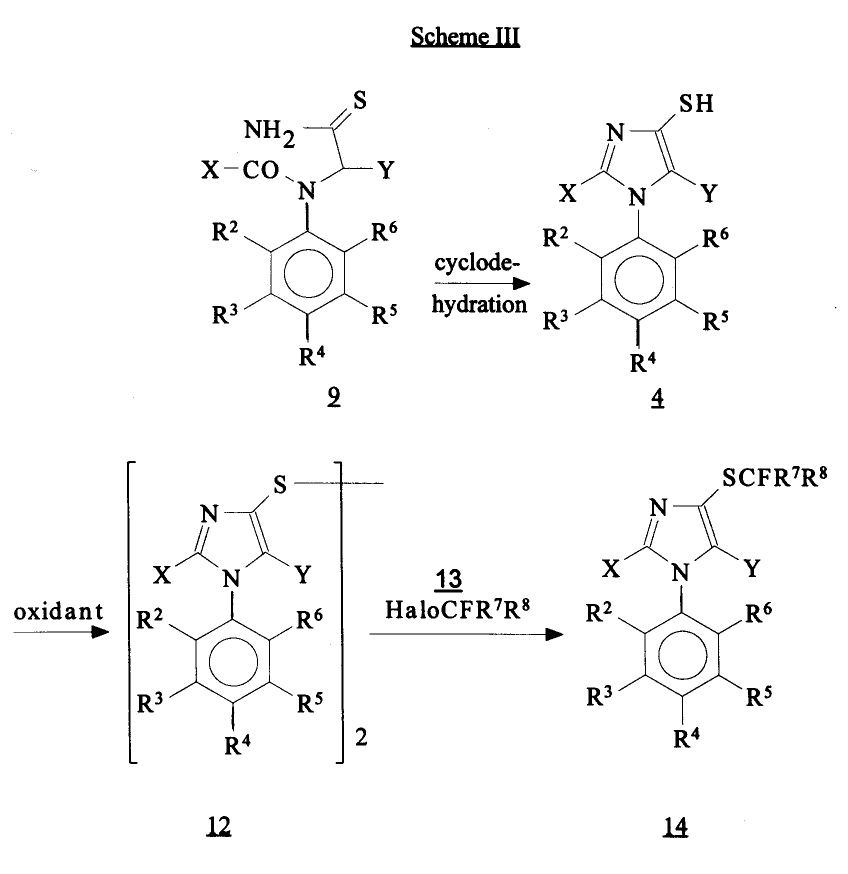
In a first step, we describe hereafter the manufacturing process of a new compound of the present invention, of the general formula Ia, namely of formula 4, starting from the novel compound of the present invention, of formula 9.

Cyclodehydration of carbothioamide methylene substituted N carbonylated amines of formula 9 is generally known in the art (J. Org.Chem. 1987, 52, 2977 and J.Org. Chem. 1989, 54, 4570), and any method similar to the described methods can be used, preferably heating in formic acid.

In a second step, the manufacturing process of a compound of formula 12, starting from the intermediate thiol of formula 4, is described.

The intermediate thiol 4 may either be oxidized in situ, without isolation, to the corresponding disulfide of the general formula 12, by the oxygen present in the solvent used for its preparation, for example by the oxygen present in the formic acid if the formic acid itself is used as solvent, or isolated as crude material and oxidized by further treatment with an oxidizing agent. The oxidizing agent may be oxygen or a hydrogen peroxyde, and then the reaction is conducted in a suitable inert solvent, such as acetonitrile, water, a mixture of both. If the oxidizing agent is a sulfoxide, such as dimethylsulfoxide or tetramethylene sulfoxide, then the sulfoxide itself is also used as solvent for the reaction. The oxidizing agent may also be bromine. Such a reaction is conveniently carried out at a reaction temperature from about 0 to about 200°C, preferably at a temperature from about 20 to about 150°C. Representative procedures - reported in J. March, "Advanced Organic Chemistry", Third Ed., 1985, Wiley-Interscience, p. 1092 and in references therein, in O. G. Lowe, J. Org. Chem., 1975, 40 , 2096, and in T. J. Wallace, J. Am. Chem. Soc., 1964, 86, 2018.

A sulfide 14 may be prepared by the reaction of the disulfide intermediate 12 and a perhaloalkane compound of the formula 13, HaloCFR⁷R⁸ , wherein Halo is a halogen, preferably Cl, Br or I, R⁷ is F, Cl or Br, and R⁸ is F, Cl, Br or a perfluoroalkyl group, with a reducing agent which can promote the formation of the free radical CFR⁷R⁸ (from HaloCFR⁷R⁸). The reducing agent is preferably chosen from a metal consisting of zinc, aluminum, cadmium, manganese or a compound with an oxide of sulfur, e. g. a dithionite or a hydroxymethylsulfinate. The alkaline dithionite, alkaline earth or the metal dithionite corresponds to the formula Mₙ(S₂O₄), in which n can be 1 or 2 depending upon the valence of the metal M. When a dithionite or a hydroxymethylsulfinate is used, a base is needed. The base can he chosen from among an alkaline hydroxide, alkali earth hydroxide, ammonia, alkylamine, triethylbenzylammonium or the salt of weak acids such as disodium phosphate, sodium metabisulfite, sodium hydrogen sulfite, sodium borate. The solvents used for the reaction are those which can solubilize the dithionite or the hydroxymethylsulfinate and the compounds 12 and 13. Useful solvents are acetonitrile, dimethylformamide, formamide, dimethylacetamide, hexamethylphosphoramide, N-methylpyrrolidone, dimethylsulfoxide or sulfolane. The reaction temperature is from about 10°C to about 200°C, preferably from about 50°C to about 150°C. Typical procedures are similar to those reported by C. Wakselman et al., J. Chem. Soc., Chem. Commun., 1991, 993 and in references therein.

The sulfides of the general formula 14, in which Y is a hydrogen atom and the other groups are as defined above, are also useful intermediates in the preparation of compounds of the general formula **III**, in which Y is a halogen atom, Z is CFR⁷R⁸, n is 0, and the other groups are as defined above. The reaction is represented by the following equation: 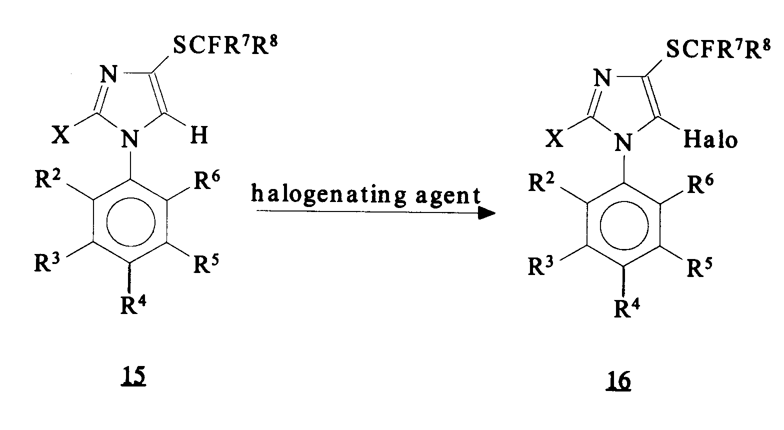
Thus a sulfide of the general formula 16, wherein Halo is a halogen atom, preferably Cl, Br or I and the other groups are as defined above, may be prepared by halogenating a sulfide intermediate of the general formula 15, with a halogenating agent, such as chlorine or bromine, N-chlorosuccinimide or N-bromosuccinimide. The reaction is conducted in a inert aprotic solvent, such as a chlorinated hydrocarbon, a ether, acetonitrile, or in a biphasic reaction medium which may be either a liquid-solid or a liquid-liquid system. When the biphasic reaction medium is a liquid-solid system, the liquid phase may be a aliphatic or aromatic hydrocarbon which may be halogenated. Referred organic solvents are chlorinated aromatic hydrocarbon such as chlorobenzene. The solid phase is an inorganic base such as a hydroxyde or carbonate or hydrogen carbonate of alkaline or earth-alkaline metal. Referred bases are carbonate and hydrogen carbonates of alkaline metals such as sodium carbonate and sodium hydrogen carbonate. When the biphasic reaction medium is a liquid-liquid system, one of the liquid phase is an organic phase which is mainly a aliphatic or aromatic hydrocarbon which may be halogenated. Referred organic solvents are chlorinated aromatic hydrocarbons such as chlorobenzene. The other liquid phase is a aqueous phase which contains a phase transfer catalyst such as a alkylammonium halide.. The reaction may be carried at between about -50°C and 150°C, preferably between about -10°C and 100°C, depending on the reactivity of the intermediate 15 and the reactivity of the halogenating agent used. Typical procedures are similar to those reported in A. R. Katritzky et al., "Comprehensive Heterocyclic Chemistry", 1984, Vol. 5, Part 4A, Ed. Pergamon Press, p. 398 and in references therein.

The compounds of the general formula **III**, in which n is 0 and the other groups are as defined above, are also useful intermediates in the preparation of compounds of the general formula **III**, in which n is 1 or 2 and the other groups are as defined above. Their general preparations and the corresponding procedures are described in the European patent application EP 0 396427 cited above.

### EXAMPLES:

The following examples are given to illustrate the invention and some modes of realisation. It is not intended to restrict the invention to these particular examples.

### Examples relating to the compounds of general formula Ia :

The following examples further illustrate some of the preparation of preferred and representative non-limiting compounds of the general formulae (III) and (Ia) of this invention, and the intermediates and processes thereto. Details of typical methods of synthesis utilized in the preparation of intermediates and compounds of the invention are specifically provided below for compounds of **Examples 1, 4, 5, 11, 12, 13, 24, 31, 34, 42, 43 and 44.**

The other compounds were prepared using similar methods of synthesis or modifications thereof of the detailed procedures as applicable to a given compound. These compound Examples are listed in **Tables I (Examples 2 and 3), II (Examples 6 to 10), III (Examples 14 to 23), IV (Examples 25 to 30), V (Examples 32 and 33), VI (Examples 35 to 41) and VII (Examples 45 to 47).**

Reporting melting points for compounds represent the average value of a observed melting point range determined for a compound or furthermore represent the average value of a number of separate melting point determinations. Additionally, one or more spectroscopic analysis (IR, NMR, GC/MS, etc.) has been performed when needed for characterization and confirmation of the chemical structure.

The above methods or processes of synthesis are not to be construed as limiting and therefore, compounds of the present invention as well as intermediates ad starting materials (particularly the anilines), can be prepared by application or adaptation of synthesis methods, which are apparent to one skilled in the art, and are commonly known, used or described in the chemical literature. In this regard, it is understood that, for example, the sequence of the synthetic chemical steps may be performed in a different order as appropriate, suitable protecting groups may be employed, and substituent groups may be incorporated when convenient. In the description of process methods, when symbols appearing in a formula are not specifically defined, it is also to be understood that they are "as herein before defined" in accordance with the first definition of each symbol in this specification.

In an overall/global manner the foregoing Methods of synthesis may be represented by the following processes of the invention which are described as follows:

### Examples 1-12

### (Process SCHEME I)

### Preparation of N-cyanoalkylaniline intermediates of the general formula 2.

### a) Example 1: preparation of N-(2,4-dichlorophenyl)aminoacetonitrile.

To a solution of 2,4-dichloroaniline (20g, 0.123 mole) in NN-dimethylformamide (170mL) was added bromoacetonitrile (43mL, 0.615 mole), 2,6-lutidine (29mL,0.246 mole), and sodium iodide (18.5g, 0.123 mole). The dark solution was heated to ca 100°C for 2h. Then the reaction was cooled to room temperature, concentrated and the residue dissolved in ethyl acetate (150mL). The resultant solution was washed with water (250mL) and the aqueous layer back extracted with ethyl acetate (125mL). The combined organic layers were washed with brine (125mL), dried (Na₂SO₄), filtered, concentrated and chromatographed through silica gel (32-62 microns) contained in a 350mL glass fritted funnel using hexane:ethyl acetate/90:10 (1L), followed by 80:20 (1L). Isolation gave an oil that was crystallized from ethyl acetate:hexane/1:4 (45mL). The resultant solid was rinsed with hexane to give 23.2g (94% yield) of the desired product, mp 76.0-77.0°C.

The compounds of **Examples 2-3** were prepared using the procedure of Example 1. These compound Examples are listed in **Table I.**

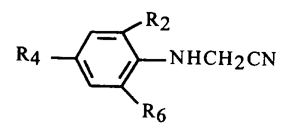

**Table I**

| additional compounds synthesized according to procedure of Example 1. | | | | | |
|---|---|---|---|---|---|
| Example | R₂ | R₄ | R₆ | yield (%) | mp (°C) |
| 2 | Cl | F | Cl | 69 | 90-95 |
| 3 | F | F | H | 63 | 61-65 |

### b) Example 4: preparation of N-(4-fluorophenyl)-aminoacetonitrile.

To a 1L reaction flask equipped with an addition funnel and reflux condenser was added water (325mL), formaldehyde sodium bisulfite addition compound(130.3g, 0.97 mole), and 4-fluoroaniline (61.4mL, 0.648 mole). After heating the heterogeneous mixture 30 minutes at 85°C, a solution of potassium cyanide (46.4g, 0.71 mole) in water (70mL) was added over a five minute period. The temperature was increased to 100°C and the reaction stirred for 1 hour. The reaction, which contained a suspended oil, was cooled to room temperature, transferred to a separatory funnel and the layers were partitioned. The aqueous layer was extracted with dichloromethane (2 x 300mL) and the combined organics were washed with water (1 x 300mL). The organic layer was dried over sodium sulfate, filtered and concentrated to give 62g of an oil that was dissolved in diethyl ether (75mL) and placed in the refrigerator overnight. The resulting dense solid was rinsed with hexane, ground to a powder and rinsed twice more with hexane to give 44.3g (46% yield) of N-(4-fluorophenyl)-aminoacetonitrile, mp 46.5-47.5°C, which was used without further purification.

### c) Example 5: preparation of 2-(4-fluorophenylamino)-propionitrile.

To a solution of 4-fluoroaniline (8.6mL, 90.0 mmol) in ethyl alcohol (100mL) was added lactonitrile (8.4mL, 116mmol). The reaction was heated to reflux for 3h, then cooled to room temperature and concentrated. The residue, which solidified upon standing, was washed with water (2 x 50mL), then dissolved in dichloromethane (100mL) and dried over sodium sulfate, filtered and concentrated. The resultant solid was recrystallized from dichloromethane to give 9.9g (67% yield) of a light brown solid of 2-(4-fluorophenylamino)-propionitrile, mp 74-78°C.

The compounds of **Examples 6-10** were prepared using the procedure of Example 5. These compound Examples are listed in **Table II.**

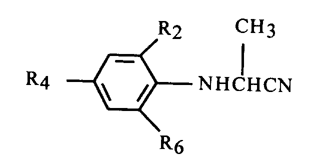

**Table II**

| additional compounds synthesized according to procedure of Example 5. | | | | | |
|---|---|---|---|---|---|
| Example | R₂ | R₄ | R₆ | yield (%) | mp (°C) |
| 6 | Cl | F | Cl | 58 | 85-87 |
| 7 | F | F | H | 79 | 56-58 |
| 8 | Cl | Cl | Cl | 33 | 110-111 |
| 9 | CH₃ | F | H | 99 | oil |
| 10 | H | Cl | H | 99 | |

### d) Example 11: preparation of N-(2-chloro-4-fluorophenyl)-aminoacetonitrile.

To a solution of N-(4-fluorophenyl)-aminoacetonitrile (0.8g, 5.33 mmol), prepared as in Example 4, in acetonitrile, (20mL) was added N-chlorosuccinimide (0.71g, 5.33 mmol). The resulting solution was heated to 45°C overnight. After 16h, additional N-chlorosuccinimide (0.11g, 0.80 mmol) was added and the heating continued. Three hours later, the reaction was concentrated and the residue taken up in dichloromethane (50mL) and washed with water (50mL) followed by sodium bicarbonate (aqueous saturated, 50mL). The organic layer was dried over sodium sulfate, filtered, and concentrated. Chromatographic purification, through silica gel (32-62 microns) contained in a 60mL glass fritted funnel using hexane:ethyl acetate/90:10 (400mL), afforded 0.5g (51% yield) of N-(2-chloro-4-fluorophenyl)-aminoacetonitrile, mp 76.5-78.0°C.

### e) Example 12: preparation of 2-(2,6-dichloro-4-fluorophenylamino)-propionitrile.

The intermediate was prepared according to procedure of Example 11, except that the stoichiometry of N-chlorosuccinimide was increased to 2 equivalents. A solid of 2-(2,6-dichloro-4-fluorophenylamino)-propionitrile was obtained in 55% yield, mp 85-87°C.

### Examples 13-23

### (Process SCHEME I)

### Preparation of carbothioamide intermediates of the general formula 3.

### Example 13: preparation of N-(2,4-dichlorophenyl)-aminothioacetamide.

To a solution of N-(2,4-dichlorophenyl)-aminoacetonitrile (23.2g, 0.115 mole), prepared as in Example 1, in ethyl alcohol (225 mL), was added triethylamine (33.8 ml, 0.24 mole). Hydrogen sulfide (16g, 0.46 mole) was added, at room temperature, in 3 portions via gas dispersion tube. After the last portion of hydrogen sulfide was added (at 5 hours), nitrogen was bubbled through the reaction for 2 hours. The reaction was then concentrated to a dark foam which was partially redissolved in dichloromethane (50mL) and placed in the refrigerator for 30 minutes. The solid was then collected and rinsed with hexane (3 x 25 mL) to give 21.5g (79% yield) of a dark purple powder of N-(2,4-dichlorophenyl)-aminothioacetamide, mp 142-144°C decomposed.

The compounds of **Examples 14-23** were prepared using the procedure of Example 13. These compound Examples are listed in **Table III.**

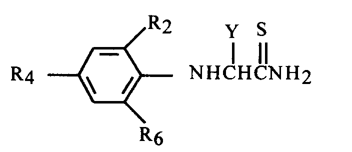

**Table III**

| additional compounds synthesized according to procedure of Example 13. | | | | | | |
|---|---|---|---|---|---|---|
| Example | Y | R₂ | R₄ | R₆ | yield (%) | mp (°C) |
| 14 | H | Cl | F | Cl | 99 | 104-105 |
| 15 | CH₃ | Cl | F | Cl | 99 | 123-125 |
| 16 | CH₃ | Cl | Cl | Cl | 80 | 77-80 |
| 17 | H | F | F | H | 87 | 160-162 |
| 18 | CH₃ | F | F | H | 99 | 125-134 |
| 19 | H | H | F | H | 68 | 110 dec. |
| 20 | H | Cl | F | H | 99 | |
| 21 | CH₃ | CH₃ | F | H | 59 | |
| 22 | CH₃ | H | Cl | H | 89 | |
| 23 | CH₃ | Cl | F | F | 56 | |

### Examples 24-33

### (Process SCHEME III)

### Preparation of sulfide compounds of the invention and disulfide intermediates of the general formula 4 and 12.

### a) Example 24: preparation of 4,4'-dithiobis[1-(4-fluorophenyl)-imidazole].

N-(4-fluorophenyl)-aminothioacetamide (4.0g, 0.022 mole), prepared as in Example 19, was dissolved in triethylorthoformate (70 mL). The solution was heated to 100°C for 3h, cooled to room temperature, and concentrated to give an oily residue (ca 8g) of a mixture of 1-(4-fluorophenyl)-4-mercaptoimidazole and 4,4'-dithiobis[1-(4-fluorophenyl)-imidazole].

The residue was dissolved (without further purification) in dimethylsulfoxide (60mL) and stirred at room temperature for 16h. The dark solution was then concentrated, diluted with ethyl acetate (100mL) and a solid was filtered off to give 2.8g (66% yield) of 4,4'-dithiobis[1-(4-fluorophenyl)-imidazole], mp 211.5-212.5°C.

The compounds of **Examples 25-30** were prepared using the procedure of Example 24. These compound Examples are listed in **Table IV.**

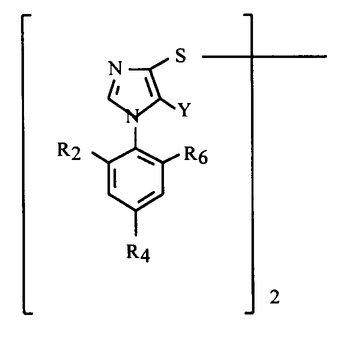

**Table IV**

| additional sulfide and disulfide compounds synthesized according to procedure of Example 24. | | | | | | |
|---|---|---|---|---|---|---|
| Example | Y | R₂ | R₄ | R₆ | yield (%) | mp (°C) |
| 25 | H | Cl | F | Cl | 94 | 236-239 |
| 26 | H | F | F | H | 99 | |
| 27 | H | Cl | F | H | 99 | oil |
| 28 | CH₃ | CH₃ | F | H | 99 | oil |
| 29 | CH₃ | H | Cl | H | 99 | |
| 30 | CH₃ | Cl | F | F | 98 | oil |

### b) Example 31: preparation of 4,4'-dithiobis[1-(2,6-dichloro-4-fluorophenyl)-5-methyl imidazole].

2-(2,6-dichloro-4-fluorophenylamino)thiopropionamide (43g, 161mmol), prepared as in Example 15, was dissolved in an excess of triethylorthoformate (300mL) and stirred at 130-140°C for a total of 30h. The reaction mixture was filtered to remove precipitated product, which was washed with ethyl acetate, giving 28.1g (68% yield) of 4,4'-dithiobis[1-(2,6-dichloro-4-fluorophenyl)-5-methyl imidazole], mp 318°C decomp.

The compounds of **Examples 32-33** were prepared using the procedure of Example 31. These compound Examples are listed in **Table V.**

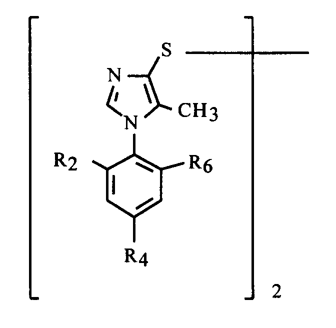

**Table V**

| additional compounds synthesized according to procedure of Example 31. | | | | | |
|---|---|---|---|---|---|
| Examp le | R₂ | R₄ | R₆ | yield (%) | mp (°C) |
| 32 | Cl | Cl | Cl | 58 | 297 dec. |
| 33 | F | F | H | 72 | |

### Examples 34-43

### (Process SCHEME III)

### Preparation of sulfides of the general formula 14.

### a) Example 34:preparation of 1-(4-fluorophenyl)-4-dichlorofluoromethylsulfenyl imidazole.

To a 250mL Fisher-Porter bottle was added 4,4'-dithiobis[1-(4-fluorophenyl)-imidazole] (2.8g, 7.25mmol), prepared as in Example 24, in NN-dimethylformamide (90mL). As nitrogen was bubbled through the heterogeneous mixture, the flask was cooled to ca -20°C. After 30 min., the nitrogen purge was pulled from the liquid and sodium formate (2.0g, 29.0mmol) was added followed by sulfur dioxide (2.1mL, 47.9mmol) and fluorotrichloromethane (2.7mL, 29.0mmol). The pressure bottle was sealed and the reaction warmed to room temperature and stirred overnight at a resultant pressure of 9 psi. After 22h, the cloudy orange mixture was concentrated, diluted with ethyl acetate (150mL) and washed with water (100mL). The organic layer was dried over sodium sulfate, filtered, concentrated. Chromatographic purification, through silica gel (32-62 microns) contained in a 150mL glass fritted funnel using hexane:ethyl acetate/70:30 (1L), afforded 2.7g (63% yield) of 1-(4-fluorophenyl)-4-dichlorofluoromethylsulfenyl imidazole, mp 112.0-113.0°C.

The compounds of **Examples 35-41** were prepared using the procedure of Example 34. These compound Examples are listed in **Table VI.**

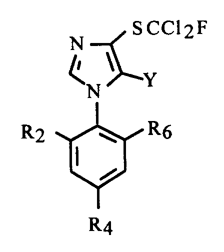

**Table VI**

| additional compounds synthesized according to procedure of Example 34. | | | | | | |
|---|---|---|---|---|---|---|
| Example | Y | R₂ | R₄ | R₆ | yield (%) | mp (°C) |
| 35 | H | Cl | F | Cl | 53 | 68-70 |
| 36 | H | F | F | H | 83 | oil |
| 37 | CH₃ | F | F | H | 58 | oil |
| 38 | H | Cl | F | H | 56 | |
| 39 | CH₃ | CH₃ | F | H | 66 | 65-67 |
| 40 | CH₃ | H | Cl | H | 52 | 85-87 |
| 41 | CH₃ | Cl | F | F | 26 | 80-81 |

### b) Example 42:preparation of 1-(2,4,6-trichlorophenyl)-5-methyl-4-dichlorofluoromethylsulfenyl imidazole.

The compound was prepared according to procedure of Example 34, except that the reaction was heated in 1-methyl-2-pyrrolidinone at 75°C for 4h. A solid of 1-(2,4,6-trichlorophenyl)-5-methyl-4-dichlorofluoro-methylsulfenyl imidazole was obtained in 43% yield, mp 102.5-103°C.

### c) Example 43:preparation of 1-(2,6-dichloro-4-fluorophenyl)-5-methyl-4-dichlorofluoromethylsulfenyl imidazole.

The compound was prepared according to procedure of Example 34, except with these changes in stoichiometry: 8 equivalents of sodium formate; 36 equivalents of sulfur dioxide; 40 equivalents of fluorotrichloromethane. The reaction was heated in 1-methyl-2-pyrrolidinone at 65°C for 7h. A solid of 1-(2,6-dichloro-4-fluorophenyl)-5-methyl-4-dichlorofluoromethylsulfenyl imidazole was obtained in 43% yield, mp 72.5-73.5°C.

### Examples 44-47

### Preparation of sulfides of the general formula 16.

### Example 44:preparation of 1-(2,6-dichloro-4-fluorophenyl)-5-bromo-4-dichlorofluoromethylsulfenyl imidazole.

To a 4L three neck reaction flask equipped with mechanical stirrer and nitrogen inlet was added 1-(2,6-dichloro-4-fluorophenyl)-4-dichlorofluoromethylsulfenyl imidazole (117.1g, 0.32mol), prepared as in Example 35, in acetonitrile (2.5L). To this solution was added N-bromosuccinimide (57.2g, 1.0eq) and the reaction stirred at room temperature overnight (ca 16hr). The reaction was concentrated, taken up in dichloromethane (ca 100mL) and filtered. The mother liquor was coated onto silica gel, evaporated, added to a bed of ca 2 kg silica gel 60 (32-62 microns) and eluted with 10L hexane:ethyl acetate/90:10. Purification gave 118.6g (83% yield) of a viscous orange oil of 1-(2,6-dichloro-4-fluorophenyl)-5-bromo-4-dichlorofluoromethylsulfenyl imidazole.

The compounds of **Examples 45-47** were prepared using the procedure of Example 44. These compound Examples are listed in **Table VII.**

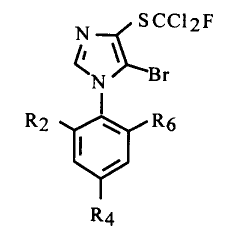

**Table VII**

| additional compounds synthesized according to procedure of Example 44. | | | | | |
|---|---|---|---|---|---|
| Example | R₂ | R₄ | R₆ | yield (%) | mp (°C) |
| 45 | F | F | H | 28 | 52-56 |
| 46 | Cl | F | H | 24 | 65-67 |
| 47 | H | F | H | 79 | 97.0-97.5 |

### Examples relating to the compounds of general formula Ib :

### Example 48 :

316 g (10.5 moles) of polyoxymethylene were added progressively at 10°C to 551 g (11.25 moles) of sodium cyanide in 2 l of water, followed by the progressive addition of 1563 g (8.85 moles) of benzenesulphonyl chloride. The reaction mixture was further stirred at 10°C. To the previous reaction mixture were added 8 l of methylene chloride ; then were introduced at 10°C 1561 g(7.5 moles) of N-formyl-2,6-dichloro-4-fluoro aniline and 485 g (1.64 moles) of tetrabutylammonium chloride. The solution was stirred at 10°C during 20 min., then mixed with 975 ml (9.75 moles) of an aqueous solution of sodium hydroxyde at 30%. After stirring 1 hour at 10°C, 2 l water and 2 l methylene chloride were added. The organic layer was washed with water and concentrated. Crystallisation in the mixture cyclohexane/isopropanol (75/25) supplied 1702 g (89 % yield) of N-cyanomethyl, N-formyl 2,6-dichloro-4-fluoroaniline.

This product was characterized by proton nuclear magnetic resonance spectrometry in dimethylsulfoxid the aromatic protons were found to give two doublets at a chemical shift of 7.71 and 7.76 ppm, each corresponding to a different conformer, with a coupling constant of 8.5 Hz.

### Example 49 :

Cyanomethyl acetate was prepared according to the method described in Synth. Commun. 2, 215 (1972). A mixture of 2.78 g (10 millimole) of tetrabutylammonium chloride and 1 g (10 millimole) of cyanomethyl acetate was dissolved in 10 ml of dichloromethane. To the reaction mixture were added 2 g (9.6 millimoles) of N-formyl-2,6 dichloro-4-fluoroaniline and 3 ml (12 millimoles) of a 4 mole/l sodium hydroxyde aqueous solution. After stirring for 1 hour at room temperature, N-cyanomethyl-N-formyl-2,6-dichloro-4-fluoroaniline was obtained.

### Example 50 :

To a solution of 6.16 g (29.6 millimoles) of N-formyl-2,6-dichloro-4-fluoroaniline in 30 ml of dichloromethane were added at room temperature 30 ml of water, 1.66 g of tetrabutylammonium chloride (5.9 millimole), 1.5 g (37.5 millimole) of sodium hydroxyde and finally a solution of 2.1 ml (33.0 millimole) of chloroacetonitrile in 3 ml of dichloromethane. After stirring for 1 hour at room temperature, the organic layer was washed with water, dried, concentrated, and washed with 100 ml of hexane to afford 5.7 g (78%) of N-cyanomethyl-N-formyl-2,6-dichloro-4-fluoroaniline.

### Example 51 :

To 1513 g (6.12 moles) of the end product of example 1, in 15 l ethanol, were added at room temperature 1822 g (18 moles) of triethylamine. 510 g (15 moles) of hydrogen sulfide (gas) were introduced in this solution in 2.5 hour at 30°C. A stream of inert gas was passed through the reaction mixture for 8 hours at room temperature. Then, the mixture was concentrated, cooled down to 0° C and filtered. The solid was washed with cold (-30°C) ethyl alcohol and dried to give 1640 g of 2-[N-formyl-N-(2,6-dichloro-4-fluoro)phenyl] thionoacetamid ( 93% yield).

This product was characterized by proton nuclear magnetic resonance spectrometry in dimethylsulfoxid: the aromatic protons were found to give two doublets at a chemical shift of 7.59 and 7.66 ppm, each corresponding to a different conformer, with a coupling constant of 8.5 Hz.

## Claims

1. A compound of the general formula **I** : 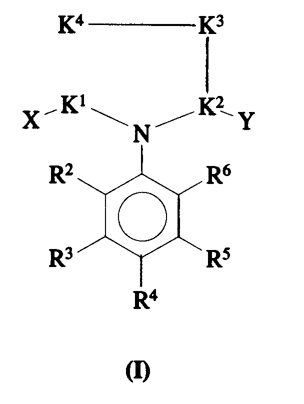 wherein :
- K¹ represents -CO-, or 〉C= ,K¹ being connected to K⁴ through the double free bond when K¹ is 〉C=
- K² represents 〉C= the double free bond being connected to K³, or K² is 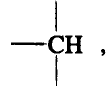 and
- K³ represents : 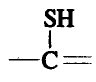 or ≡C- or -CS- , and
- K⁴ represents :
- -N= when K¹ represents 〉C= , K⁴ being then connected both with K³ and K¹ through the covalent bond
- or N≡ or NH₂-, K⁴ being then connected only to K³ through one covalent bond,
the link between K⁴ and K³ being either a monovalent bond or a trivalent bond according to the specific meaning of K⁴ and K³ respectively,
the link between K³ and K² being either a monovalent bond or a divalent bond according to the specific meaning of K³ and K² respectively,
- X, Y, R², R³, R⁴, R⁵ and R⁶ are each individually selected from : a hydrogen or a halogen atom or a cyano group or a C₁ to C₄ straight chain or branched chain alkyl, which group is unsubstituted or substituted by one or more halogen atoms, which are the same or different, up to full substitution; and
provided that R² and R⁶ are not simultaneously a methyl when:
. X and Y represent a hydrogen atom, and
. K² is 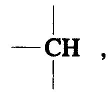 and K³ is ≡C- , and K⁴ is N≡

2. A compound according to claim 1 of formula (Ia) : 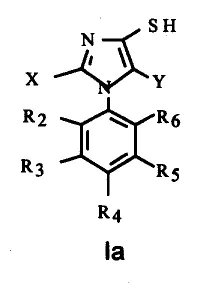 wherein :
X, Y, R², R³, R⁵ and R⁶ are each individually selected from: a hydrogen or a halogen atom or a C₁ to C₄ straight chain or branched chain alkyl, which group is unsubstituted or substituted by one or more halogen atoms, which are the same or different, up to full substitution; and
R⁴ is selected from: a hydrogen or a halogen atom or a cyano group or a unsubstituted C₁ to C₄ alkyl or C₁ to C₄ alkyl substituted by one or more halogen atoms, which are the same or different, up to full substitution.

3. A compound according to claim 1 of formula (Ib): 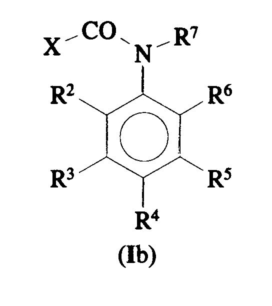 wherein:
X is a hydrogen atom or a C₁ to C₄ straight chain or branched chain alkyl group, which group is unsubstituted or substituted by one or more halogen atoms which are the same or different, up to full substitution of the alkyl moiety ; and
R², R³, R⁴, R⁵, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety ; and
R⁷ represents a group -CHY-CN or -CHY-CS-NH₂, in which Y is a hydrogen atom or a C₁ to C₄ straight chain or branched chain alkyl group
provided that R² ad R⁶ are not simultaneously a methyl when X and R⁷ represent, respectively, a hydrogen atom and a CH₂-CN group.

4. A compound according to claim 1 of formula (II): 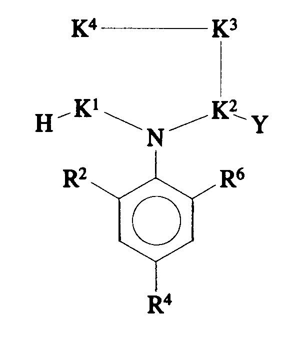 wherein :
- K¹ represents -CO-, or 〉C= ,K¹ being connected to K⁴ through the double free bond when K¹ is 〉C=
- K² represents 〉C= the double free bond being connected to K³, or K² is 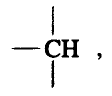 and
- K³ represents : 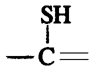 or ≡C- or -CS- , and
- K⁴ represents :
- -N= when K¹ represent 〉C= , K⁴ being then connected both with K³ and K¹ through the covalent bond ,
- or N≡ or NH₂-, K⁴ being then connected only to K³ through one covalent bond,
the link between K⁴ and K³ being either a monovalent bond or a trivalent bond according to the specific meaning of K⁴ and K³ respectively,
the link between K³ and K² being either a monovalent bond or a divalent bond according to the specific meaning of K³ and K² respectively,
Y is a hydrogen atom or methyl; and
R², R⁴, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety ; and
provided that R² and R⁶ are not simultaneously a methyl when:
. Y represents a hydrogen atom, and
. K² is 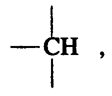 and K³ is ≡C- , and K⁴ is N≡

5. A compound according to one of the claims 1, 2 and 4, of formula (IIa): 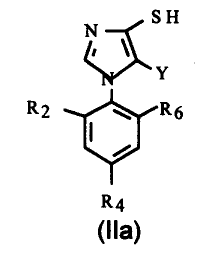 wherein:
Y is a hydrogen atom or methyl; and
R² and R⁶ are a hydrogen or a halogen atom or methyl; and
R⁴ is a halogen atom.

6. A compound according to one of the claims 1, 3 and 4, of formula (IIb) : 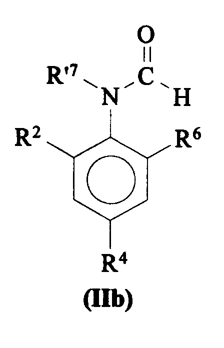 wherein :
R², R⁴, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety ;
R'⁷ represents a group -CH₂-CN or -CH₂-CS-NH₂ ;
provided that R² and R⁶ are not simultaneously a methyl when R'⁷ represents a CH₂-CN group.

7. A compound according to claim 6, of formula **IIb** wherein :
R² and R⁶ are a halogen or a hydrogen, and
R⁴ is a halogen.

8. A compound according to claim 5 which is 1-(4-fluorophenyl)-4-mercaptoimidazole.

9. A compound according to claim 5 which is 1-(2,6-dichloro-4-fluorophenyl)-4-mercaptoimidazole.

10. A compound according to claim 5 which is 1-(2,4-difluorophenyl)-4-mercaptoimidazole.

11. A compound according to claim 5 which is 1-(2-chloro-4-fluorophenyl)-4-mercaptoimidazole.

12. A compound according to claim 5 which is 1-(2-methyl-4-fluorophenyl)-4-mercapto-5-methylimidazole.

13. A compound according to claim 5 which is 1-(4-chlorophenyl)-4-mercapto-5-methylimidazole.

14. A compound according to claim 5 which is 1 -(2-chloro-4,6-difluorophenyl)-4-mercapto-5-methylimidazole.

15. A compound according to claim 5 which is 1-(2,6-dichloro-4-fluorophenyl)-4-mercapto-5-methylimidazole.

16. A compound according to claim 5 which is 1-(2,4,6-trichlorophenyl)-4-mercapto-5-methylimidazole.

17. A compound according to claim 5 which is 1-(2,4-diflurophenyl)-4-mercapto-5-methylimidazole.

18. A compound according to claim 7 which is N-cyanomethyl, N-formyl 2,6-dichloro-4-fluoroaniline or 2-[N-formyl-N-(2,6-dichloro-4-fluoro)phenyl] thionoacetamid

19. A compound of formula 7 : 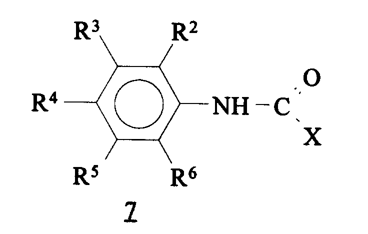 wherein X and R² to R⁶ are as defined in formula **Ib** in claim 3

20. A process for the production of a compound of formula Ia 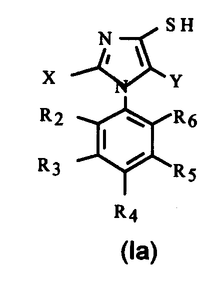 wherein:
X, Y, R², R³, R⁵ and R⁶ are each individually selected from: a hydrogen or a halogen atom or a C₁ to C₄ straight chain or branched chain alkyl which group is unsubstituted or substituted by one or more halogen atoms, which are the same or different, up to full substitution; and
R⁴ selected from: a hydrogen or a halogen atom or a cyano group or a unsubstituted C₁ to C₄ alkyl or C₁ to C₄ alkyl substituted by one or more halogen atoms, which are the same or different, up to full substitution,
which comprises the step of reacting a carbothioamide compound of the formula 3: 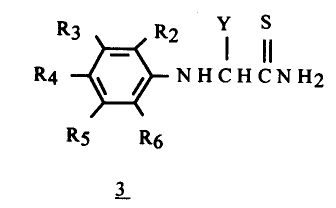 wherein Y and R² to R⁶ are as defined for Formula Ia with an alkyl orthoformate of the formula 5
XC(0R)₃ (5)
wherein R is alkyl of 1 to 4 carbon atoms and X is as defined for formula Ia, optionally in the presence of a inert solvent, at a temperature of between about 20 to about 200°C.

21. A process according to claim 20 wherein the optional solvent is toluene, chlorobenzene, dimethylformamide, dimethylsulfoxide or preferably excess alkyl orthoformate.

22. A process for the production of a compound of the general formula **Ib**: 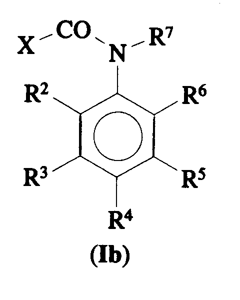 wherein :
X is a hydrogen atom or a C₁ to C₄ straight chain or branched chain alkyl group, which group is unsubstituted or substituted by one or more halogen atoms which are the same or different, up to full substitution of the alkyl moiety ; and
R², R³, R⁴, R⁵, R⁶ represent a hydrogen or a halogen atom or a C₁ to C₄ straight chain or branched chain alkyl group, which group is unsubstituted or substituted by one or more halogen atoms which are the same or different, up to full substitution of the alkyl moiety ; and
R⁷ represents a group -CHY-CN or -CHY-CS-NH₂, in which Y is a hydrogen atom or a C₁ to C₄ straight chain or branched chain alkyl group ;
which comprises the step of reacting a N-carbonylated aniline of formula 7 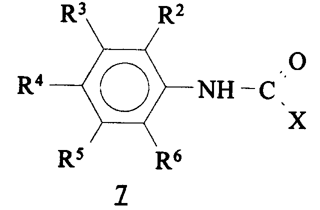 wherein X and R² to R⁶ are as defined in claim 3 with a compound of formula (11):
Σ-CHY-CN (11)
wherein:
Σ is a halogen , hydroxyde, alkylcarbonyloxy, haloalkylcarbonyloxy, arylcarbonyloxy, alkylsulfonyloxy, haloalkylsulfonyloxy, arylsulfonyloxy, preferably phenylsulfonyloxy ;
Y has the same definition as in formula **Ib** in claim 3

23. A process according to claim 22 for the production of a compound of the general formula 8 : 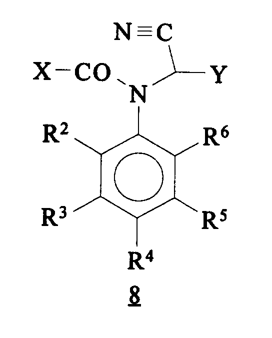 wherein the substituents are as defined in claim 3

24. A process according to claim 22 for the production of a compound of the general formula 9: 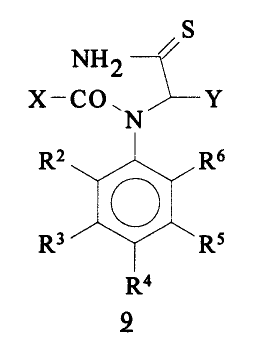 wherein the substituents are as defined in general formula **Ib** in claim 3

25. A process according to one of the claims 22 to 24 wherein the reaction of the compound 7 with the compound 11 is carried out in a biphasic reaction medium comprising a organic liquid phase and an aqueous liquid phase

26. A process according to claim 25 wherein the organic liquid phase is a aliphatic or aromatic hydrocarbon which may be halogenated, or a derivative of such compounds having a oxygen atom therein which forms a ether bond

27. A process according to claim 25 wherein the aqueous liquid phase comprises a water soluble base ad a phase transfert catalyst

28. A process according to claim 27 wherein the water soluble base is a hydroxide or carbonate of alkaline or earth-alkaline metal

29. A process according to claim 27 wherein the phase transfert catalyst is a alkylammonium halide

30. A process according to one of the claims 22 to 29 wherein the reaction of the compound 7 with the compound 11 is carried out at a temperature between -10 and 50 °C, preferably between 5 ad 20 °C

31. A process for the production of a compound of claim 2 which comprises the step of reacting a compound of formula 9 : 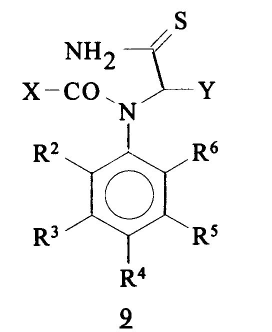 wherein the substituents are as defined in claim 3 with a cyclodehydration agent
